Europäisches Patentamt

European Patent Office

Office européen des brevets

(11) Numéro de publication: **0 116 793**
**B1**

(12) **FASCICULE DE BREVET EUROPEEN**

(45) Date de publication du fascicule du brevet: 06.04.88

(21) Numéro de dépôt: 83402365.7

(22) Date de dépôt: 07.12.83

(51) Int. Cl.⁴: **G 01 N 33/569,**
G 01 N 33/559

(54) Procédé de détection immunobactériologique de germes pathogènes dans des milieux biologiques contaminés.

(30) Priorité: 09.12.82 FR 8220632

(43) Date de publication de la demande:
29.08.84 Bulletin 84/35

(45) Mention de la délivrance du brevet:
06.04.88 Bulletin 88/14

(84) Etats contractants désignés:
AT BE CH DE FR GB IT LI LU NL SE

(56) Documents cités:
FR-A-1 573 936
FR-A-2 262 533
FR-A-2 334 106
FR-A-2 390 502
FR-A-2 463 807

(73) Titulaire: **INSTITUT PASTEUR Fondation
reconnue d'utilité publique
28 rue du Docteur Roux
F-75724 Paris Cédex 15 (FR)**

(72) Inventeur: **Dodin, André
29 Boulevard Edgar Quinet
F-75014 Paris (FR)**
Inventeur: **Avrameas, Eustrate
1 Rue Sarasate
F-75015 Paris (FR)**
Inventeur: **Goud, Bruno
42 Rue de Cronstadt
F-75015 Paris (FR)**
Inventeur: **Guillou, Michel
6 avenue Jean-Lurcat
F-78340 Les Clayes-Sous-Bois (FR)**
Inventeur: **Nicolas, Renée
14 Avenue Pierre Brossolette
F-92246 Malakoff (FR)**

(74) Mandataire: **Ores, Irène et al
CABINET ORES 6, Avenue de Messine
F-75008 Paris (FR)**

Courier Press, Leamington Spa, England.

EP 0 116 793 B1

## Description

La présente invention est relative à un nouveau procédé de détection immunobactériologique de germes pathogènes possédant des déterminants antigéniques spécifiques tels que notamment la fraction 1 + 2 définie dans le Brevet français ANVAR N° 2 215 945 dans des milieux biologiques contaminés, et notamment à un procédé de détection immunobactériologique de germes pathogènes possédant une fraction 1 + 2, tels que Vibrio cholerae, Salmonella typhimurium, Malleomyces mallei, Pseudomonas pseudomallei, dans des milieux biologiques contaminés, et notamment dans les selles, le sang, l'urine, l'eau, les excrétions de toutes sortes.

Par déterminants antigéniques on entend toutes fractions provenant d'un agent pathogène susceptibles d'induire un anticorps polyspécifique ou monoclonal capable de reconnaître ledit agent pathogène.

Les fractions antigéniques 1 + 2 sont connues par le Brevet français ANVAR N° 2 215 945 du 2 février 1973 et les premier et deuxième Certificats d'Addition à celui-ci n° 2 262 533 du 1er mars et N° 2 388 049 du 22 Avril 1977.

Comme on le sait, les fractions antigéniques 1 + 2 sont des fractions antigéniques obtenues à partir de cultures de Vibrio cholerae, Salmonella typhimurium, Malleomyces mallei ou Pseudomonas mallei; ces fractions sont caractérisées par le fait qu'elles correspondent au premier pic sur le diagramme d'absorption dans l'ultraviolet à 280 nm et par le fait que lorsq'elles sont opposées à un immunsérum produit chez un animal à partir de la même culture, elles donnent la bande ou le couple de bandes de précipitation le plus proche du puits d'antigène dans l'épreuve de diffusion en milieu gélosé et dans l'épreuve d'analyse immuno-électrophorétique.

La fraction antigénique 1 + 2 se définit également, conformément au Brevet français 2 215 945, comme étant La première fraction lipido-protéinique sortant d'une colonne de chromatographie de dextrane en grains de leur production endocellulaire.

On connait, d'autre part, par le Brevet français N° 2 334 106 au nom de l'INSTITUT PASTEUR, um gel magnétique utilisable pour des dosages immunoenzymatiques constitués par un gel d'acrylamide/agarose contenant des particules magnétiques, couplé avec une protéine telle qu'un antigène ou un anticorps par l'intermédiaire d'un agent de couplage tel que le glutaraldéhyde, un tel gel couplé à un antigène ou à un anticorps étant utilisé pour le dosage immuno-enzymatique de l'anticorps ou de l'antigène correspondant présent dans un liquide biologique, par incubation de ce gel avec le liquide biologique, suivie de plusieurs lavages du gel avec séparation de ce dernier à l'aide d'un champ magnétique, puis incubation du gel avec des anticorps ou des antigènes marqués à l'aide d'une enzyme couramment utilisée en dosage immuno-enzymatique telle que la glucose-oxydase ou la peroxydase, et mesure de l'activité enzymatique du gel par lecture de la densité optique.

Le Brevet français N° 2 463 807 au nom de L'INSTITUT PASTEUR et de l'ANVAR propose un procédé de fixation de molécules biologiques protéiques telles que des antigènes, des anticorps, des enzymes, des virus au des cellules, sur des particules de polymères magnétiques vinyl-aromatiques, par adsorption, par simple mise en contact des molécules biologiques dans un tampon approprié, avec des billes des polymères magnétiques précités, pendant 1 heure à 37°C et une nuit à 4°C.

Par ailleurs, la demande de brevet français INSTITUT PASTEUR et C.N.R.S. N° 2 514 367 du 8 Octobre 1981 fait connaître un procédé de détection de germes pathogènes dans des fluides, et notamment dans l'eau d'alimentation, par filtration sur un immunoadsorbant constitué par un support insoluble, et plus particulièrement un polymère insoluble tel qu'un gel, notamment gel de polyacrylamide et/ou d'agarose, sur lequel sont fixés par liaison chimique, des anticorps spécifiques du germe pathogène dont on souhaite déterminer la présence dans un fluide, en particulier des anticorps anti-fraction Ch 1 + 2 de Vibrion cholerae, des anticorps anti-Salmonella thyphimurium, des anticorps anti-virus polyomyélitique du type I et II, anti-Escherichia coli, anti-virus de l'encéphalomyocardite, cette filtration étant suivie de la caractérisation des facteurs antigéniques retenus par l'immunoadsorbant, laquelle caractérisation est réalisée par des moyens classiques tels que dilution du gel et culture sur gélose en milieu liquide sélectif ou caractérisation biochimique et sérologique du germe (agglutination).

La présente invention a pour but d'isoler le germe pathogène du milieu biologique contaminé, directement sur des billes de gel magnétique sur lesquelles est fixé un anticorps, en le concentrant à l'aide de ces billes en des quantités très supérieures aux faibles concentrations en germes initialement présentes dans un milieu biologique. Ceci permet de diagnostiquer non seulement la maladie, mais la présence de germes pathogènes chez un porteur de ces germes en faibles quantités et la caractérisation du germe pathogène en mois de 48 heures en immunodiffusion, ainsi que de diagnostiquer la présence éventuelle de plusieurs germes pathogènes dans un même milieu biologique, entraînant ainsi une économie notable de milieu à tester et de temps puisque plusieurs germes peuvent être détectés simultanément grâce au procédé qui fait l'objet de la présente invention, qui, en outre, ne nécessite pas la mise en oeuvre de milieux de culture sélectifs.

La présente invention a pour objet un procédé de détection immunobactériologique de germes pathogènes possédant des déterminants antigéniques spécifiques, tels que notamment la fraction antigénique 1 + 2, dans des milieux biologiques contaminés, à l'aide d'un gel magnétique sur lequel sont fixés des anticorps anti-déterminants antigéniques spécifiques, lequel procédé est caractérisé en ce qu'on met en contact un milieu biologique supposé contaminé par un germe pathogène possédant ledit déterminant antigénique spécifique, tel que par exemple la fraction 1 + 2 avec des billes ou analogues dudit gel magnétique, pendant un temps suffisant pour permettre la fixation sur le gel, des germes contenus dans le milieu biologique, en ce qu'on retire les billes ou analogues de gel magnétique du mélange, par des moyens magnétiques, et en ce qu'on les ensemence sur un milieu gélosé approprié sur lequel elles sont incubées pendant une durée et à une température appropriées, après quoi on forme par tous moyens appropriés dans la gélose, à proximité des billes magnétiques recouvertes de colonies de cultures, incrustées dans la gélose, des zones contenant du sérum anti-germe pathogène à détecter, approprié, en ce qu'on lyse les germes au moyen d'agents de lyse appropriés, en ce qu'on incube le lysat avec le sérum pendant un temps approprié pour permettre à la réaction sérologique anticorps-antigènes de se produire par immunodiffusion sur la gélose et en ce qu'on dénombre par lecture directe, grâce à la réaction antigène-anticorps sur la gélose, la quantité des germes contenus dans le milieu biologique.

Selon un mode de réalisation avantageux du procédé de détection immunobactériologique conforme à la présente invention, applique à la détection simultanée de plusieurs germes pathogènes dans un milieu biologique, les puits creusés dans la gélose sont remplis de sérums anti-germe pathogène différents.

Selon un autre mode de réalisation avantageux du procédé conforme à l'invention, les anticorps anti-déterminants antigéniques spécifiques fixés sur les billes ou analogues de gel magnétique, sont des anticorps anti-fraction 1 + 2 provenant d'un sérum hyperimmun anti-fraction 1 + 2 obtenu conformément au Brevet français N° 2 215 945 ou à l'une de ses Additions N° 2 262 533 ou 2 388 049, par extraction des IgG dudit sérum hyperimmun.

Selon une modalité avantageuse de ce mode de réalisation, pour l'application du procédé conforme à l'invention à la détection simultanée de plusieurs germes pathogènes dans un milieu biologique, des séries de billes ou analogues de gel magnétique sur chacune desquelles sont fixés des anticorps anti-fraction 1 + 2 provenant de cultures de chacun des germes pathogènes à détecter, sont mises en contact avec le milieu biologique.

Conformément à l'invention, le sérum anti-germe pathogène introduit dans les puits creusés dans la gélose est choisi dans le groupe qui comprend les sérums spécifiques de germes anti-fraction 1 + 2, les sérums polyvalents anti-germes pathogènes possédant une fraction 1 + 2, les sérums anti-toxine du germe pathogène, les sérums anti-sous-unité A ou B desdits germes pathogènes.

Selon un mode de réalisation avantageux du procédé conforme à l'invention, le milieu biologique auquel le procédé est appliqué est choisi dans le groupe qui comprend les selles, le sang, l'urine, les excrétions de toutes sortes, l'eau.

Selon un autre mode de réalisation avantageux du procédé conforme à l'invention, les moyens magnétiques utilisés pour retirer les billes ou analogues de gel magnétique du milieu biologique après incubation avec ce dernier, sont constitués par une tige aimantée.

Selon encore un autre mode de réalisation avantageux du procédé conforme à l'invention, l'agent de lyse des germes en culture sur les billes incrustées dans la gélose, est un agent de lyse chimique tel que le toluène, un Tween® (qui est le nom générique d'agents mouillants obtenus par condensation d'un détergent connu sous la marque "Span"®, formé d'esters d'acides gras et de sorbitol, avec l'oxyde d'éthylène) ou le glucose à forte concentration, notamment, ou un agent de lyse enzymatique tel que du Complément, par exemple.

Selon un mode de réalisation avantageux du procédé conforme à l'invention, les billes ou analogues de gel magnétique fixées sur les moyens magnétiques sont soumises avant d'être ensemencées sur la gélose, à plusieurs lavages.

Selon un autre mode de réalisation avantageux du procédé conforme à l'invention, les billes ou analogues de gel magnétique fixées sur les moyens magnétiques, sont détachées de ces derniers pour être ensemencées sur la gélose, par action d'un champ magnétique.

Conformément à l'invention, la durée du contact entre les billes de gel magnétique auxquelles sont fixés des anticorps anti-déterminants spécifiques, est d'environ 2 heures, à la température ambiante; la durée de la culture sur gélose des germes fixés sur ces billes de gel magnétique est de l'ordre de 14 à 18 heures, à 37°C ± 1°C et la durée de la réaction entre les anticorps anti-fraction 1 + 2 et les antigènes de sérum contenu dans les puits, par immunodiffusion, est de l'ordre de 14 à 18 heures à la température ambiante.

La présente invention a également pour objet un coffret ou "kit" prêt à l'emploi pour la mise en oeuvre du procédé de détection immunobactériologique de germes pathogènes possédant des déterminants antigéniques tels que la fraction 1 + 2, dans des milieux biologiques contaminés, défini dans ce qui précède, lequel coffret est caractérisé en ce qu'il comprend (dans le cas notamment de la fraction 1 + 2):

— un réceptacle contenant une quantité appropriée de billes de gel magnétique sur lesquelles est fixé un anticorps anti-fraction 1 + 2,

— une tige ou analogue aimantée,

— une boîte de culture, telle qu'une boîte de Pétri, notamment, dont la face extérieure du fond est pourvue en son centre d'un aimant de forte puissance,

— un réceptacle contenant une quantité appropriée de milieu gélosé pour la culture de germes pathogènes possédant une fraction 1 + 2,

— un réceptacle contenant une quantité appropriée de sérum anti-germe pathogène à détecter,

— un réceptacle contenant une quentité appropriée d'agents de lyse des cultures de germes pathogènes sur gélose,

— un réceptacle contenant une quentité appropriée d'eau de lavage physiologique tamponnée.

Selon un mode de réalisation avantageux du coffret ou kit prêt à l'emploi, utilisable pour la détection simultanée de plusieurs germes pathogènes dans un milieu biologique, celui-ci comprend plusieurs réceptacles contenant chacun une quantité appropriée de billes de gel magnétique auxquelles sont fixés des anticorps anti-fraction 1 + 2 différents et plusieurs réceptacles contenant chacun un sérum anti-germe pathogène à détecter correspondant à chacun des anticorps, différents.

Selon un autre mode de réalisation avantageux du coffret ou kit prêt à l'emploi conforme à l'invention, la tige ou analogue aimantée est une tige aimantée enrobée. de "Teflon" (Marque déposée) ou d'un autre matériau isolant analogue.

Selon encore un autre mode de réalisation avantageux du coffret ou kit prêt à l'emploi conforme à l'invention, la tige ou analogue aimantée est constituée par un dispositif aimanté tel que le dispositif qui fait l'objet du brevet français N° 2 554 016 au nom de l'INSTITUT PASTEUR.

Selon un mode de mise en oeuvre préféré de l'invention, la détection immunobactériologique de germes pathogènes possédant une fraction 1 + 2, dans un milieu biologique contaminé, est réalisée de la façon suivante:

1) On utilise des billes de gel magnétique "Act. Magnogel® AcA 44" comprenant 4% de polyacrylamide, 4% d'agarose, 7% de $Fe_2(SO_4)_3$, de 60 à 140 µm de diamètre et présentant une capacité de couplage pour les IgG de 2 à 4 mg/ml. ("Magnogel" est une marque déposée).

2) On fixe des IgG extraites d'un sérum hyperimmun anti-fraction 1 + 2 obtenu par la technique d'immunisation d'OUDIN, en injectant à un lapin une fraction antigénique 1 + 2 obtenue conformément aux dispositions du Brevet français ANVAR N° 2 215 945 ou de l'une de ses Certificats d'Addition N° 2 262 533 ou 2 388 049, c'est-à-dire soit par séparation de germes gram négatifs mis en culture, dans des milieux de culture appropriés, par centrifugation, extraction des fractions endogéniques endocellulaires par lyse des germes dans des conditions stériles (soit par congélation-décongélation, soit par ultrasons), puis séparation de la fraction antigénique 1 + 2 par chromatographie sur gel; soit par lyse des germes par la méthode de Gallut, et purification de la fraction antigénique 1 + 2 par l'acide chloracétique, soit par autoclavage du bouillon de culture, suivi d'une centrifugation et d'une dialyse du surnageant contre de l'eau distillée.

La fixation des IgG sur les billes de gel magnétique est réalisée soit par couplage chimique, à l'aide de glutaraldéhyde, conformément au brevet français INSTITUT PASTEUR N° 2 334 106, soit par adsorption, conformément au brevet français INSTITUT PASTEUR et ANVAR N° 2 463 807.

Les IgG fixées sur les billes de gel magnétique sont soit identiques sur toutes les billes si on vise à détecter la présence d'un germe pathogène, soit différentes si on vise à détecter plusieurs germes pathogènes dans un même milieu biologique.

3) Les billes de gel magnétiques sur lesquelles sont fixés 2 à 4% d'anticorps anti-fraction 1 + 2, sont mises en contact pendant environ 2 heures à la temperature du laboratoire, avec un milieu biologique contaminé en solution tel que de l'eau, des selles, du sang, de l'urine ou d'autres excrétions.

4) On plonge une tige aimantée convenablement isolée, d'environ 12 mm de diamètre, telle qu'une tige aimantée enrobée de "Téflon" (marque déposée) ou le dispositif aimanté conforme au brevet INSTITUT PASTEUR N° 2 554 016, dans la solution, avec agitation manuelle lente; les billes magnétiques viennent se fixer sur la tige. On lave 3 fois dans 250 ml d'eau physiologique tamponnée à pH 7,4.

5) Après le troisième lavage, les billes magnétiques concentrées à la face inférieure de la tige sont ensemencées sur un milieu gélosé du commerce, par exemple un milieu gélosé PASTEUR à 1,5% ("Agar Noble" Difco) ou un milieu MULLER-HINTON, contenu dans une boîte de Pétri sous laquelle est placé un aimant de forte puissance disposé sensiblement dans l'axe de la boîte; la tige aimantée chargée de billes est placée au centre de la boîte de Pétri, au contact de la gélose; sous l'action du champ magnétique émis par l'aimant placé sous la boîte, les billes se détachent de la tige et s'incrustent dans la gélose sur laquelle elles forment une tâche de 1 cm de diamètre environ. La boîte de Pétri est incubée pendant 14 à 18 heures environ à 37°C ± 1°C. Il est également possible avec une anse de prélever, si on le désire, des germes de ces colonies en vue d'une bactériologie classique.

6) A la fin de la période d'incubation, on creuse à environ 1 cm de la tache de billes magnétiques recouvertes de colonies, 3 ou 4 trous à l'aide d'un emporte-pièce stérile. On coule une microgoutte de gélose au fond de chacun des puits pour en colmater le fond, puis on remplit les puits de sérum spécifique du germe pathogène anti-fraction 1 + 2, ou de sérum polyvalent anti-germe pathogène à détecter, ou de sérum anti-toxine complète du germe pathogène à détecter ou de sérum anti-sous-unité A ou B de la toxine du germe pathogène à détecter. En variante, on remplit chacun des puits de sérums correspondants à des germes pathogènes différents si les billes magnétiques portent elles-mêmes des anticorps anti-fraction 1 + 2 différents, pour permettre la détection de plusieurs germes pathogènes dans un même milieu biologique.

Les sérums mis en oeuvre sont obtenus par la méthode de OUDIN, à partir de cultures de germes pathogènes gramnégatifs possédant une fraction antigénique 1 + 2, tels que Vibrio cholerae, Salmonella typhimurium, Malleomyces mallei, Pseudomonas mallei (ou Bacille de Whitmore), Pseudomonas pseudomallei, Legionellea pneumophila, Escherichia coli, Toxine LT, notamment, de même que les IgG fixées sur les billes de gel magnétique sont extraites de sérums des germes pathogènes précités.

7. On dépose sur les billes magnétiques recouvertes de colonies, une goutte d'un agent de lyse chimique tel que du toluène ou un Tween — ou du glucose si les IgG fixées sont des IgG anti-fraction 1 + 2 de Vibrio cholerae —, ou d'un agent de lyse enzymatique tel que de Complément.

8) La boîte de PETRI est fermée pour éviter toute évaporation de son contenu et maintenue pendant 14 à 18 heures à la température ambiante, pour permettre à la réaction sérologique antigènes-anticorps de se produire par immunodiffusion dans la gélose.

A l'issue de cette période d'incubation, les antigènes du germe pathogène se révèlent sur la gélose par une ou plusieurs bandes de précipitation contre le sérum correspondant: c'est ainsi que les antigènes de Vibrio cholerae se révèlent par une bande unique de précipitation contre le sérum anti-fraction Ch 1 + 2 spécifique, par 4 ou 5 bandes contre le sérum polyvalent anticholérique. Les souches non-toxinogènes ne présentent pas de bandes anti-toxine ou anti-sous-unité toxique. Les souches toxinogènes se révèlent par une bande de précipitation contre le sérum anti-toxine ou les sérums anti-sous-unités A et B de la toxine.

Le procédé de détection de germes pathogènes conforme à l'invention présente le très grand avantage, par rapport à la méthode préconisée par l'Organisation Mondiale de la Santé qui ne permet que d'identifier le germe pathogène, sans permettre de reconnaître son caractère toxinogène, de permettre de détecter un germe toxinogène. Dans le cas de Vibrio cholerae par exemple, le procédé conforme à l'invention permet de dénombrer des quantités de vibrions allant de $10^2$/100 ml (cas des porteurs de germes) à $10^6$/ml (cas des malades) et dans le cas d'Escherichia coli les quantités dénommées peuvent être de $10^2$/1000 ml (porteurs de germes).

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention.

Il doit être bien entendu, toutefois, que ces exemples de mise en oeuvre, sont donnés uniquement à titre d'illustration de l'objet de l'invention, dont ils ne constituent en aucune manière une limitation.

### Exemple 1

Détection de germes de Vibrio cholerae chez un porteur de germe

1) On part de selles supposées contaminées par Vibrio cholerae El Tor sérotype Ogawa; on réalise une suspension de selles à 10% sur agitateur: 10 g de selles pour 100 millilitres d'eau. On ajoute à cette suspension 1 ml de billes "Magnogel" contenant 2 mg par ml d'anticorps anti-fraction Ch 1 + 2.

On laisse en contact pendant 2 heures à la température ambiante.

2) On plonge une tige aimantée enrobée de "Teflon", de 12 mm de diamètre — ou la tige aimantée du dispositif conforme au brevet français INSTITUT PASTEUR N° 2 554 016 dans la suspension, avec agitation lente; les billes se fixent sur la tige; on lave 3 fois dans 250 ml d'eau physiologique tamponnée à pH 7,5.

3) On place la tige à la face inférieure de laquelle sont concentrées les billes, au contact d'un milieu gélosé PASTEUR à 1,5% ("Agar Noble" Difco) contenu dans une boîte de Pétri sous laquelle est placé un aimant de forte puissance; la tige est placée au centre de la boîte, dans le même axe que l'aimant. Les billes se détachent et s'incrustent dans la gélose pour former une tache de 1 cm de diamètre.

La boîte de Pétri est incubée pendant 18 heures à 37°C.

4) A la fin de cette période d'incubation, on creuse dans la gélose 4 trous en croix à l'aide d'un emporte-pièce stérile, à environ 1 cm de la tache de billes magnétiques recouvertes de colonies. On coule une microgoutte de gélose au fond des puits pour les colmater et on remplit ceux-ci avec du sérum anti-toxine cholérique complète.

5) On dépose sur les billes magnétiques recouvertes de colonies, une goutte de toluène qui lyse immédiatement les germes.

6) On ferme la boîte de Pétri, et on l'incube pendant 18 heures à la température ambiante.

Au bout de ce temps les antigènes de la souche toxinogène de Vibrio cholerae se révèlent par une bande de précipitation contre le sérum anti-toxine.

### Exemple 2

Détection de germes de Vibrio cholerae chez un malade

En procédant comme décrit à l'Exemple 1 ci-dessus et en plaçant dans deux puits de sérum anti-fraction Ch 1 + 2 et dans les deux autres puits du sérum anti-toxine cholérique complète, on a dénombré $10^6$ vibrions/ml de solution de selles et déterminé qu'il s'agit d'une souche toxinogène, alors qu'en soumettant un échantillon des mêmes selles à la méthode O.M.S. on a pu identifier le germe, mais non son caractère toxinogène.

### Exemple 3

Détection de germes de bacilles de Whitmore chez un porteur de germe

1) Le produit biologique, 20 g de crottin de cheval supposé contaminé par des bacilles de Whitmore, est dilué par de l'eau physiologique pour obtenir un volume d'environ 50 ml; on ajoute 0,20 ml de billes magnétiques + anticorps anti-Whitmore type I. On laisse au contact 1 à 2 heures à la temperature du laboratoire.

2) Passé ce délai, on prélève avec une tige aimantée les billes qui sont ensuite déposées au centre d'un milieu gélosé nutritif, (par exemple milieu de Mueller-Hinton) contenu dans une boîte de Pétri, par exemple. On place à l'étuve pendant 18 heures à 37°C.

3) On creuse, à 8 mm de la pastille de culture sur billes, des puits dans lesquels seront déposés les sérums spécifiques anti-Whitmore Type I.

4) On lyse les germes de la pastille par une goutte de toluène.

5) Après 10 à 12 heures est visible une bande de précipitation antisérum bacille de Whitmore-anticorps Whitmore type I si le crottin renfermait des bacilles de Whitmore.

6) Il est possible de remplacer les puits anticorps par de petits disques de papier (type antibiotique) imprégnés du sérum spécifique.

7) Résultats.

On obtient, aussi bien pour le type I que pour le type II, une concentration importante des germes, sensiblement du même ordre qu'à l'Exemple 1.

L'immunsérum spécifique ne précipite que contre les antigènes du bacille de Whitmore de type I ou de type II, en sorte que même si la spécificité de fixation n'est pas absolue (on a détecté des Proteus fixés sur les billes), elle n'est cependant pas gênante pour la détection.

### Exemple 4
Détection de germes de Salmonella thyphimurium

Des essais ont été menés sur des selles contaminées expérimentalement, en mettant en oeuvre le mode opératoire décrit à l'Exemple 1, avec des bacilles de Salmonella thyphimurium. La concentration des Salmonelles sur gélose comme décrit à l'Exemple 1 a été du même ordre que pour V. cholerae.

L'antigène commun aux différentes types de Salmonelles (A, B, C, D, E) qui s'est révélé sur gélose, est extrait après repérage et identification par diagramme de double diffusion en gélose, sur une colonne de "Sephadex G200" (marque déposée) et utilisé pour obtenir, par injection à des lapins, le sérum spécifique.

Le diagnostic est effectué par précipitation contre les sérums spécifiques.

### Exemple 5
Détection de germes d'Escherichia coli

Les essais ont été effectués sur des selles contaminées expérimentalement, en procédant comme décrit à l'Exemple 1. Les mêmes bons résultats de concentration ont été obtenus: $10^2$ Escherichia coli dans 1000 ml d'eau se fixent sur les billes de gel magnétique; de même, les résultats dans la spécificité de la précipitation par les sérums sont aussi bons que pour V. Cholerae.

Ainsi que cela ressort de ce qui précède, le procédé de détection conforme à l'invention permet de diagnostiquer la présence de germes pathogènes en moins de 48 heures, alors qu'il faut 4 jours au minimum pour réaliser la même détection par voie bactériologique. En outre, le procédé de détection nécessite un matériel très réduit et son prix de revient est faible. Les quantités de milieu biologique qu'il requiert sont petites, de même que les quantités de réactifs mises en oeuvre. De plus, à partir d'un seul échantillon de milieu biologique, il permet d'identifier plusieurs germes pathogènes éventuellement présents dans celui-ci, en utilisant des billes magnétiques sur lesquelles sont couplés des anticorps différents selon le germe à détecter et des sérums différents correspondants placés dans les puits creusés dans la gélose.

### Revendications

1. Procédé de détection immunobactériologique de germes pathogènes possédant des déterminants antigéniques spécifiques, dans des milieux biologiques contaminés, à l'aide d'un gel magnétique sur lequel sont fixés des anticorps anti-déterminants antigéniques spécifiques, lequel procédé est caractérisé en ce qu'on met en contact un milieu biologique supposé contaminé par un germe pathogène possédant un déterminant antigénique spécifique, avec des billes ou analogues dudit gel magnétique, pendant un temps suffisant pour permettre la fixation sur le gel, des germes contenus dans le milieu biologique, en ce qu'on retire les billes de gel magnétique du mélange, par des moyens magnétiques, et en ce qu'on les ensemence sur un milieu gélosé approprié sur lequel elles sont incubées pendant une durée et à une température appropriées, après quoi on forme par tous moyens appropriés, dans la gélose, à proximité des billes magnétiques recouvertes de colonies de culture, incrustées dans la gélose, des zones contenant du sérum anti-germe pathogène à détecter, approprié, en ce qu'on lyse les germes au moyen d'agents de lyse appropriés, en ce qu'on incube le lysat avec le sérum, pendant un temps approprié pour permettre à la réaction sérologique anticorps-antigènes de se produire par immunodiffusion sur la gélose et en ce qu'on dénombre par lecture directe, grâce à la réaction antigènes-anticorps sur la gélose, la quantité de germes contenus dans le milieu biologique.

2. Procédé selon la revendication 1, caractérisé en ce que le déterminant antigénique spécifique est constitué par la fraction antigénique 1 + 2, qui est un fraction obtenue à partir de cultures de Vibrio cholerae, Salmonella typhimurium, Malleomyces mallei ou Pseudomonas mallei et qui est caractérisée par le fait qu'elle correspond au premier pic sur le diagramme d'absorption dans l'ultra-violet à 280 nm et par le fait que lorsqu'elle est opposée à un immunsérum produit chez un animal à partir de la même culture, elle donne la bande ou le couple de bandes de précipitation le plus proche du puits d'antigène dans l'épreuve de diffusion en milieu gélosé et dans l'épreuve d'analyse immuno-électrophorétique.

3. Procédé selon la revendication 1 ou la revendication 2, caractérisé en ce que les zones contenant du sérum anti-germe pathogène à détecter, approprié, sont formées en creusant dans la gélose, des puits que l'on remplit dudit sérum.

4. Procédé selon la revendication 1 ou la revendication 2, caractérisé en ce que les zones contenant du sérum anti-germe pathogène, à détecter, approprié sont formées en disposant à proximité des billes magnétiques recouvertes de colonies de culture, incrustées dans la gélose, des petits disques de papier imprégnés dudit sérum.

5. Procédé selon l'une quelconque des revendications 1 à 4, appliqué à la détection simultanée de plusieurs germes pathogènes, caractérisé en ce que les zones contenant du sérum anti-germe pathogène à détecter, formées dans la gélose contiennent des sérums anti-germe pathogène différents.

6. Procédé selon l'une quelconque des revendications 1 à 2, caractérisé en ce que les anticorps anti-déterminants antigéniques spécifiques fixés sur les billes ou analogues de gel magnétique, sont des anticorps anti-déterminants antigèniques spécifiques provenant d'un sérum hyperimmun antidéterminant antigénique spécifique, obtenus par extraction des IgG dudit sérum hyperimmun.

7. Procédé selon la revendication 6, pour l'application à la détection simultanée de plusieurs germes pathogènes dans un milieu biologique, caractérisé en ce que des séries de billes ou analogues de gel magnétique sur chacune desquelles sont fixés des anticorps anti-déterminants spécifiques provenant de cultures de chacun des germes pathogènes à détecter, sont mises en contact avec le milieu biologique.

8. Procédé selon la revendication 5, caractérisé en ce que le serum anti-germe pathogène contenu dans les zones formées dans la gélose, est choisi dans le groupe qui comprend les sérums spécifiques de germes anti-déterminants antigéniques spécifiques, les sérums polyvalents anti-germes pathogènes possédant un déterminant antigénique spécifique, les sérums anti-toxine du germe pathogène, les sérums anti-sous-unité A ou B des toxines desdits germes pathogènes.

9. Procédé selon l'une quelconque des revendications 1 à 8, caractérisé en ce que le milieu biologique auquel le procédé est appliqué, est choisi dans le groupe qui comprend les selles, le sang, l'urine, les excrétions de toutes sortes l'eau.

10. Procédé selon la revendication 1, caractérisé en ce que les moyens magnétiques utilisés pour retirer les billes ou analogues de gel magnétique au milieu biologique après incubation avec ce dernier, sont constitués par une tige aimantée, avantageusement convenablement isolée.

11. Procédé selon la revendication 1, caractérisé en ce que l'agent de lyse des germes en culture sur les billes incrustées dans la gélose est un agent de lyse chimique pris dans le groupe qui comprend notamment le toluène, un produit de condensation d'un détergent formé d'esters d'acides gras et de sorbitol, avec l'oxyde d'éthylène (Span®), ou le glucose.

12. Procédé selon la revendication 1, caractérisé en ce que l'agent de lyse des germes en culture sur les billes incrustées dans la gélose, est un agent de lyse enzymatique tel que le Complément, notamment.

13. Procédé selon l'une quelconque des revendications 1 à 10, caractérisé en ce que les billes ou analogues de gel magnétique fixées sur les moyens magnétiques sont soumises, avant d'être ensemencées sur la gélose à plusieurs lavages.

14. Procédé selon l'une quelconque des revendications 1 à 10, caractérisé en ce que les billes ou analogues de gel magnétique fixées sur les moyens magnétiques, sont détachées de ces derniers pour être ensemencées sur la gélose, par action d'un champ magnétique.

15. Procédé selon l'une quelconque des revendications 1 à 14, caractérisé en ce que la durée du contact entre les billes de gel magnétique sur lesquels sont fixés des anticorps anti-déterminants antigéniques spécifiques, est d'environ 2 heures, à la température ambiante.

16. Procédé selon l'une quelconque des revendications 1 à 14, caractérisé en ce que la durée de la culture sur gélose des germes fixés sur les billes de gel magnétique, est de l'ordre de 14 à 18, heures, à 37°C ± 1°C.

17. Procédé selon l'une quelconque des revendications 1 à 16, caractérisé en ce que la durée de la réaction entre les anticorps anti-déterminants antigéniques spécifiques et les antigènes du sérum contenu dans les puits, par immunodiffusion, est de l'ordre de 14 à 18 heures à la température ambiante.

18. Coffret ou "kit" prêt à l'emploi pour la mise en oeuvre du procédé selon l'une quelconque des revendications 1 à 17, caractérisé en ce qu'il comprend:

— un réceptacle contenant une quantité appropriée de billes de gel magnétique sur lesquelles est fixé un anticorps anti-déterminant antigénique spécifique,

— une tige ou analogue aimantée, convenablement isolée,

— une boîte de culture, telle qu'une boîte de Pétri, notamment, dont la face extérieure du fond est pourvue en son centre d'un aimant de forte puissance,

— un réceptacle contenant une quantité appropriée de milieu gélosé pour la culture de germes pathogènes possédant un déterminant antigénique spécifique,

— un réceptacle contenant une quantité appropriée de sérum anti-germe pathogène à détecter,

— un réceptacle contenant une quantité appropriée d'agents de lyse des cultures de germes pathogènes sur gélose,

— un réceptacle contenant une quantité appropriée d'eau de lavage physiologique tamponnée.

19. Coffret selon la revendication 18, caractérisé en ce qu'il comprend comme anticorps anti-déterminants antigèniques spécifiques, un anticorps anti-fraction 1 + 2, et comme déterminant antigénique spécifique, la fraction 1 + 2 définie dans la revendication 2.

20. Coffret ou kit prêt à l'emploi selon les revendications 18 et 19 utilisable pour la détection simultanée de plusieurs germes pathogènes dans un milieu biologique, caractérisé en ce qu'il comprend plusieurs réceptacles contenant chacun une quantité appropriée de billes de gel magnétique sur lesquelles sont fixés des anticorps antidéterminants antigéniques spécifiques différents et plusieurs réceptacles contenant chacun un sérum antigerme pathogène à détecter correspondant à chacun des anticorps différents.

**Patentansprüche**

1. Verfahren zur immunobakteriologischen Bestimmung pathogener Keime in biologisch kontaminertem Milieu, die spezifische antigene Determinanten besitzen, mit Hilfe eines magnetischen Gels, auf welchem spezifische antigene Antideterminanten-Antikörper fixiert sind, dadurch gekennzeichnet, daß man ein durch einen pathogenen Keim, der eine spezifische antigene Determinante besitzt, vermutlich kontaminertes biologisches Milieu mit Kugeln oder Analogen aus dem genannten magnetischen Gel während eines Zeitraums, der ausreicht, um die Fixierung der in dem biologischen Milieu enthaltenen Keime auf dem Gel zu erlauben, in Kontakt bringt, daß man durch magnetische Mittel die Kugeln aus magnetischem Gel aus dem Gemisch entfernt und daß man sie auf ein geeignetes Agar-Agar-Milieu einimpft, auf dem sie während einer geeigneten Zeitdauer und bei geeigneter Temperatur inkubiert werden, wonach in dem Agar-Agar durch jegliche geeignete Maßnahmen bzw. Mittel in der Nähe der magnetischen Kugeln, die aus Kulturkolonien wiedergewonnen und in dem Agar-Agar eingelegt worden sind, Zonen gebildet werden, die geeignetes, zu bestimmendes Anti-pathogene-Keime-Serum enthalten, daß man die Keime mittels geeigneter Lysierungsmittel lysiert, daß man das Lysat über einen Zeitraum, der dazu geeignet ist, die serologische Antikörper-Antigen-Reaktion durch Immunodiffusion auf dem Agar-Agar ablaufen zu lassen, mit dem Serum inkubiert und daß man durch direkte Ablesung, dank der Antigen-Antikörper-Reaktion auf dem Agar-Agar, die Menge der in dem biologischen Milieu enthaltenen Keime zählt.

2. Verfahren nach Anspruch 1, dadurch gekennzeichnet, daß die spezifische antigene Determinante aus der antigenen Fraktion 1 + 2 besteht, die eine Fraktion ist, welche ausgehend von Kulturen von Vibrio cholerae, Salmonella typhimurium, Nalleomyces mallei oder Pseudomonas mallei erhalten wird und die dadurch gekennzeichnet ist, daß sie dem ersten Peak auf dem Absorptionsdiagramm im Ultraviolettbereich bei 280 nm entspricht und sie, während sie einem Immunserum, das bei einem Tier, ausgehend von der gleichen Kultur, erzeugt wird, entgegenwirkt, das Präzipitationsband bzw. das Paar von Präzipitationsbändern ergibt, welches bzw. welche der Antigenquelle in der Diffusionsprüfung in Agar-Agar-Milieu und in der immuno-elektrophoretischen Analysenprüfung am nächsten liegt bzw. liegen.

3. Verfahren nach Anspruch 1 oder 2, dadurch gekennzeichnet, daß die Zonen, die geeignetes, zu bestimmendes Anti-pathogene-Keime-Serum enthalten, durch Aushöhlen von Brunnen in dem Agar-Agar, welche man mit dem genannten Serum auffüllt, gebildet werden.

4. Verfahren nach Anspruch 1 oder 2, dadurch gekennzeichnet, daß die Zonen, die geeignetes, zu bestimmendes Anti-pathogene-Keime-Serum enthalten, durch Ablagern kleiner Papierscheiben, welche mit dem genannten Serum imprägniert sind, in der Nähe der magnetischen Kugeln, welche aus Kulturkolonien wiedergewonnen worden und in das Agar-Agar eingeimpft sind, gebildet werden.

5. Verfahren nach einem der Ansprüche 1 bis 4 zur gleichzeitigen Bestimmung mehrerer pathogener Keime, dadurch gekennzeichnet, daß die Zonen, die zu bestimmendes Anti-pathogene-Keime-Serum enthalten und in dem Agar-Agar ausgebildet sind, unterschiedliche Anti-pathogene-Keime-Sera enthalten.

6. Verfahren nach einem der Ansprüche 1 oder 2, dadurch gekennzeichnet, daß die spezifischen antigenen Antideterminanten-Antikörper, die auf den Kugeln oder Analogen aus magnetischem Gel fixiert sind, spezifische antigene Antideterminanten-Antikörper sind, die von einem hyperimmunen spezifischen Antideterminanten-Serum stammen und durch Extraktion der IgG des genannten hyperimmunen Serums erhalten werden.

7. Verfahren nach Anspruch 6 zur Anwendung bei der gleichzeitigen Bestimmung mehrer pathogener Keime in einem biologischen Milieu, dadurch gekennzeichnet, daß Serien von Kugeln oder Analogen aus magnetischem Gel, wobei auf jeder derselben spezifische Antideterminanten-Antikörper, die von Kulturen von jedem der zu bestimmenden pathogenen Keime stammen, fixiert sind, mit dem biologischen Milieu in Kontakt gebracht werden.

8. Verfahren nach Anspruch 5, dadurch gekennzeichnet, daß das Anti-pathogene-Keime-Serum, das in den im Agar-Agar gebildeten Zonen enthalten ist, aus der Gruppe ausgewählt wird, die spezifische sera von spezifischen antigenen Antideterminantenkeimen, polyvalente Antipathogene-Keine-Sera, welche eine spezifische antigene Determinante besitzen, Antioxinsera des pathogenen Keims, Anti-Untereinheit-A- oder -B-Sera der Toxine der genannten pathogenen Keime umfaßt.

9. Verfahren nach einem der Ansprüche 1 bis 8, dadurch gekennzeichnet, daß das biologische Milieu, auf das das Verfahren angewendet wird, aus der Gruppe ausgewählt wird, welche Plättchen, Blut, Urin, alle Arten von Ausscheidungen und Wasser umfaßt.

10. Verfahren nach Anspruch 1, dadurch gekennzeichnet, daß die magnetischen Mittel, die zum Entfernen der Kugeln oder Analogen aus magnetischem Gel aus dem Agar-Agar nach der Inkubation mit letzterem verwendet werden, aus einer magnetisierten Stange, die vorteilhafterweise auf geeignete Art und Weise isoliert ist, bestehen.

11. Verfahren nach Anspruch 1, dadurch gekennzeichnet, daß das Lysierungsmittel für die Keime in der Kultur auf den Kugeln, die in dem Agar-Agar eingelegt sind, ein chemisches Lysierungsmittel aus der Gruppe, die im wesentlichen Toluol, ein Kondensationsprodukt eines Reinigungsmittels, welches aus Fettsäureestern und Sorbit gebildet wird, mit Ethylenoxid (Span®) umfaßt, oder Glucose ist.

12. Verfahren nach Anspruch 1, dadurch gekennzeichnet, daß das Lysierungsmittel für die Keime in der Kultur auf den Kugeln, die in dem Agar-Agar eingelegt sind, ein enzymatisches Lysierungsmittel, wie insbesondere das Komplement, ist.

13. Verfahren nach einem der Ansprüche 1 bis 10, dadurch gekennzeichnet, daß die Kugeln oder Analogen aus magnetischem Gel, welche auf den magnetischen Mitteln fixiert sind, bevor sie auf das Agar-Agar eingeimpft werden, mehreren Waschvorgängen unterworfen werden.

14. Verfahren nach einem der Ansprüche 1 bis 10, dadurch gekennzeichnet, daß die Kugeln oder Analogen aus magnetischem Gel, welche auf den magnetischen Mitteln fixiert sind, zur Einimpfung auf dem Agar-Agar von diesen durch die Wirkung eines Magnetfelds abgelöst werden.

15. Verfahren nach einem der Ansprüche 1 bis 14, dadurch gekennzeichnet, daß die Dauer des Kontakts zwischen den Kugeln aus magnetischem Gel, auf denen spezifische antigene Antideterminanten-Antikörper fixiert sind, etwa 2 Stunden bei Umgebungstemperatur beträgt.

16. Verfahren nach einem der Ansprüche 1 bis 14, dadurch gekennzeichnet, daß die Dauer der Züchtung der Keime, die auf den Kugeln aus magnetischem Gel fixiert sind, auf dem Agar-Agar in der Größenordnung von 14 bis 18 Stunden bei 37°C ± 1°C liegt.

17. Verfahren nach einem der Ansprüche 1 bis 16, dadurch gekennzeichnet, daß die Dauer der Reaktion zwischen den spezifischen antigenen Antideterminanten-Antikörpern und den Antigenen des Serums, welches in den Brunnen enthalten ist, durch Immunodiffusion in der Größenordnung von 14 bis 18 Stunden bei Umgebungstemperatur liegt.

18. "Köfferchen" oder Kit zur Verwendung bei der Durchführungs des Verfahrens nach einem der Ansprüche 1 bis 17, dadurch gekennzeichnet, daß es folgendes umfaßt:

— einen Behälter, der eine geeignete Menge Kugeln aus magnetischem Gel, auf denen ein spezifischer antigener Antideterminanten-Antikörper fixiert ist, enthält,

— eine magnetisierte Stange bzw. einen magnetisierten Stab oder ein Analoges, zweckmäßigerweise isoliert,

— einen Züchtungsbehälter, wie eine Petrischale, insbesondere einen Behälter, dessen äußere Unterseite in ihrer Mitte einen leistungsstarken Magnet aufweist,

— einen Behälter, der eine geeignete Menge Agar-Agar-Milieu zur Züchtung pathogener Keime, die eine spezifische antigene Determinante besitzen, enthält,

— einen Behälter, der eine geeignete Menge an zu bestimmenden Anti-pathogene-Keime-Serum enthält,

— einen Behälter, der eine geeignete Menge Lysierungsmittel für die Kulturen aus pathogenen Keimen auf Agar-Agar enthält,

— einen Behälter, der eine geeignete Menge an gepuffertem physiologischem Waschwasser enthält.

19. Kit nach Anspruch 18, dadurch gekennzeichnet, daß es als spezifische antigene Antideterminanten-Antikörper einen Antifraktion-1 + 2-Antikörper und als spezifische antigene Determinante die in Anspruch 2 definierte Fraktion 1 + 2 umfaßt.

20. Köfferchen oder Kit zur Verwendung nach Anspruch 18 oder 19 zum gleichzeitigen Bestimmen mehrerer pathogener Keime in einem biologischen Milieu, dadurch gekennzeichnet, daß es mehrere Behälter, von denen jeder eine geeignete Menge Kugeln aus magnetischem Gel enthält, auf welchen verschiedene spezifische antigene Antideterminanten-Antikörper fixiert sind, und mehrere Behälter, von denen jeder ein zu bestimmendes Anti-pathogene-Keime-Serum enthält, welches jedem der verschiedenen Antikörper entspricht, umfaßt.

**Claims**

1. A method for the immunobacteriological detection of pathogenic germs possessing specific antigenic determinants, in contaminated biological media, with the aid of a magnetic gel to which antibodies directed against specific antigenic determinants are attached, which method is characterized in that a biological medium assumed to be contaminated by a pathogenic germ possessing a specific antigenic determinant is brought into contact with beads or similar bodies of the said magnetic gel for a sufficient time to enable the germs contained in the biological medium to become attached to the gel, in that the beads of magnetic gel are removed from the mixture by magnetic means, and in that they are inoculated onto an appropriate agar medium on which they are incubated for an appropriate time at an appropriate temperature, after which zones containing appropriate serum directed against the pathogenic germ to be detected are formed by any appropriate means, in the agar, near the magnetic beads covered with culture colonies and encrusted in the agar, in that the germs are lyzed by means of appropriate lyzing agents, in that the lyzate is incubated with the serum for an appropriate time to allow the antibody-antigen serological reaction to take place by immunodiffusion on the agar, and in that the number of germs contained in the biological medium is read off directly by virtue of the antigen-antibody reaction on the agar.

2. The method according to Claim 1, characterized in that the specific antigenic determinant consists of the antigenic fraction 1 + 2, which is a fraction obtained from cultures of Vibrio cholerae, Salmonella typhimurium, Nalleomyces mallei or Pseudomonas mallei, and which is characterized in that it corresponds to the first peak on the ultraviolet absorption diagram at 280 nm and in that, when it is compared with an immune serum produced in an animal from the same culture, it gives the precipitation band or pair of precipitation bands closer to the well of antigen in the diffusion test in an agar medium and in the immunoelectrophoretic analysis test.

3. The method according to Claim 1 of Claim 2, characterized in that the zones containing appropriate serum directed against the pathogenic germ to be detected are formed by hollowing out wells in the agar and filling them with the said serum.

4. The method according to Claim 1 or Claim 2, characterized in that the zones containing appropriate serum directed against the pathogenic germ to be detected are formed by placing small discs of paper, impregnated with the said serum, near the magnetic beads covered with culture colonies and encrusted in the agar.

5. The method according to any one of Claims 1 to 4, applied to the simultaneous detection of several pathogenic germs, characterized in that the zones containing serum directed against the pathogenic germ to be detected, formed in the agar, contain different sera directed against the pathogenic germ.

6. The method according to either one of Claims 1 or 2, characterized in that the antibodies directed against specific antigenic determinants which are attached to the beads or similar bodies of magnetic gel are antibodies directed against specific antigenic determinants which originate from a hyperimmune serum directed against the specific antigenic determinant, and which are obtained by extraction of the IgGs from the said hyperimmune serum.

7. The method according to Claim 6, for application to the simultaneous detection of several pathogenic germs in a biological medium, characterized in that series of beads or similar bodies of magnetic gel, each of which has attached to it antibodies directed against specific determinants which originate from cultures of each of the pathogenic germs to be detected, are brought into contact with the biological medium.

8. The method according to Claim 5, characterized in that the serum directed against the pathogenic germ which is contained in the zones formed in the agar is selected from the group comprising sera specific for germs directed against specific antigenic determinants, polyvalent sera directed against pathogenic germs possessing a specific antigenic determinant, sera directed against a toxin of the pathogenic germ, and sera directed against subunit A or B of the toxins of the said pathogenic germs.

9. The method according to any one of Claims 1 to 8, characterized in that the biological medium to which the method is applied is selected from the group comprising stools, blood, urine, all kinds of excretion, and water.

10. The method according to Claim 1, characterized in that the magnetic means used to remove the beads or similar bodies of magnetic gel from the biological medium after incubation with the latter consist of a magnetized rod which advantageously is suitably insulated.

11. The method according to Claim 1, characterized in that the agent for lyzing the germs in culture on the beads encrusted in the agar is a chemical lyzing agent selected from the group comprising, in particular, toluene, a product resulting from the condensation of a detergent, formed of fatty acid esters of sorbitol, with ethylene oxide (Span®), or glucose.

12. The method according to Claim 1, characterized in that the agent for lyzing the germs in culture on the beads encrusted in the agar is an enzymatic lyzi g agent such as, in particular, complement.

13. The method according to any one of Claims 1 to 10, characterized in that the beads or similar bodies of magnetic gel which are attached to the magnetic means are washed several times before being inoculated onto the agar.

14. The method according to any one of Claims 1 to 10, characterized in that the beads or similar bodies of magnetic gel which are attached to the magnetic means are detached from the latter, for inoculation onto the agar, by the action of a magnetic field.

15. The method according to any one of Claims 1 to 14, characterized in that the duration of the contact between the beads of magnetic gel to which antibodies directed against specific antigenic determinants are attached is about 2 hours at room temperature.

16. The method according to any one of Claims 1 to 14, characterized in that the duration of the culture, on agar, of the germs attached to the beads of magnetic gel is of the order of 14 to 18 hours at 37°C ± 1°C.

17. The method according to any one of Claims 1 to 16, characterized in that the duration of the reaction, by immunodiffusion, between the antibodies directed against specific antigenic determinants and the antigens of the serum contained in the wells is of the order of 14 to 18 hours at room temperature.

18. A ready-to-use kit for carrying out the method according to any one of Claims 1 to 17, characterized in that it comprises:
— a receptacle containing an appropriate amount of beads of magnetic gel to which an antibody directed against a specific antigenic determinant is attached,

— a magnetized rod or similar device which is suitably insulated,

— a culture dish, such as a Petri dish in particular, the outside of the bottom of the said dish being provided at its centre with a high-powered magnet,

— a receptacle containing an appropriate amount of agar medium for the culture of pathogenic germs possessing a specific antigenic determinant,

— a receptacle containing an appropriate amount of serum directed against the pathogenic germ to be detected,

— a receptacle containing an appropriate amount of agents for lyzing the cultures of pathogenic germs on agar, and

— a receptacle containing an appropriate amount of buffered physiological washing water.

19. The kit according to Claim 18, characterized in that it comprises, as the antibodies directed against specific antigenic determinants, an antibody directed against the fraction 1 + 2, and, as the specific antigenic determinant, the fraction 1 + 2 defined in Claim 2.

20. The ready-to-use kit according to Claims 18 and 19, which can be used for the simultaneous detection of several pathogenic germs in a biological medium, characterized in that it comprises several receptacles each containing an appropriate amount of beads of magnetic gel to which different antibodies directed against specific antigenic determinants are attached, and several receptacles each containing a serum directed against the pathogenic germ to be detected, corresponding to each of the different antibodies.